# EUROPÄISCHE PATENTSCHRIFT

(11) **EP 0 386 525 B1**
(45) Veröffentlichungstag und Bekanntmachung des Hinweises auf die Patenterteilung: **26.04.1995**
(21) Anmeldenummer: 90103342.3
(22) Anmeldetag: 21.02.1990
(51) Int. Cl.: A61L 27/00

(54) **Knochenersatzteil aus Glasionomerzement**
Glassionomer element for bone prosthesis
Elément de prothèse osseuse en verre ionomère

(30) Priorität: 09.03.1989 DE 3907663
(43) Veröffentlichungstag der Anmeldung: 12.09.1990
(73) Patentinhaber: THERA Patent GmbH & Co. KG Gesellschaft für industrielle Schutzrechte, D-82229 Seefeld (DE)
(72) Erfinder: Helms, Jan, Prof. Dr., 8700 Würzburg (DE); Geyer, Götz, Dr., 8700 Würzburg (DE); Wanek, Erich, Dr., D-8031 Seefeld (DE); Zöllner, Werner, Dr., D-8031 Oberpfaffenhofen (DE); Gasser, Oswald, Dr., D-8031 Seefeld (DE)
(74) Vertreter: Strehl Schübel-Hopf Groening & Partner

(56) Entgegenhaltungen:
- DE-A- 2 319 715
- US-A- 3 814 717
- US-A- 4 250 277

## Beschreibung

Aufgrund entzündlicher Prozesse, bösartiger Geschwulste oder traumatischer Ereignisse können Knochenstrukturen oder Knochenteile am menschlichen Körper zerstört werden, deren Ersatz durch entsprechende Prothesen möglich ist. Neben Metallen, die als Ersatz für hochbelastete Knochenstrukturen verwendet werden, dienen keramische Prothesen als Ersatz für weniger belastete Knochenstrukturen, z.B. im Kopfbereich. Keramische Materialien werden dort verwendet, um z.B. Teile der Gehörknöchelchenkette, der Gehörwände, oder auch des Kiefers zu rekonstruieren.

Es ist erforderlich, die prothetischen Teile den individuellen Größenverhältnissen und Gegebenheiten anzupassen. Dies ist aufgrund der schwierigen Bearbeitkeit bei keramischen Prothesen nur schwierig möglich. Das Design derartiger keramischer Prothesen muß sich daher in erster Linie an guten Möglichkeiten zur individuellen Größeneinstellung orientieren und kann weniger auf die natürlichen anatomischen Gegebenheiten Rücksicht nehmen. Eine nach diesem Gesichtspunkt konzipierte einstückige Mittelohrprothese ist z.B. in DE-C-2 905 183 beschrieben.

Die ebenfalls eingesetzten heterologen Knochentransplantate sind nicht immer verfügbar und mit Hinblick auf eine mögliche HIV-Infektion sehr problematisch geworden.

Der Erfindung liegt die Aufgabe zugrunde, Knochenstrukturen, insbesondere Knochenersatzteile, aus einem körperverträglichen Material bereitzustellen, die sich in einfacher Weise mit der Form und Größe des zu ersetzenden Knochenteils extrakorporal herstellen oder aus vorgefertigten Teilen anpassen lassen.

Die erfindungsgemäße Lösung dieser Aufgabe ist in den Ansprüchen 1, 3 und 8 angegeben. Vorteilhafte Weiterbildungen sind in den Unteransprüchen genannt.

Erfindungsgemäß bestehen die Knochenersatzstrukturen aus insbesondere kompaktem, d.h. nicht geschäumtem, Glasionomerzement und werden im Gegensatz zur bereits üblichen dentalen Verwendung von Glasionomerzementen außerhalb des Körpers hergestellt. Dabei ist es möglich, daß man das Knochenersatzteil intraoperativ aus frisch angemischtem Glasionomerzement formt, außerhalb des Körpers erhärten läßt und das erhärtete Teil anschließend implantiert. Alternativ ist es auch möglich, daß industriell vorgefertigte Formkörper, welche den zu ersetzenden Knochenstrukturen in idealisierender Form angenähert sind, durch mechanisches Bearbeiten den anatomischen Verhältnissen angepaßt werden. Im Gegensatz zur Keramik ist die Bearbeitung von Glasionomerzement-Formkörpern mit üblichen spanabhebenden Methoden problemlos.

Die erfindungsgemäßen Ersatzteile können in einfacher Weise durch plastisches Verformen der angemischten Zementmasse erhalten werden. Diese erhärtet in wenigen Minuten zu einem starren Teil, das anschließend mechanisch z.B. mit den üblichen Schleif- oder Fräsinstrumenten leicht bearbeitet werden kann. Darüberhinaus verbindet sich das erhärtete Teil chemisch mit frisch angemischtem, noch plastischem Zement, so daß die erfindungsgemäßen Ersatzteile in einfacher Weise in situ befestigt werden können. Dabei erweist es sich als vorteilhaft, daß die Glasionomerzemente eine chemische Bindung mit Hartgeweben des Körpers, wie z.B. Knochen, eingehen.

Darüberhinaus sind die erfindungsgemäßen Ersatzteile sehr biokompatibel bzw. bioaktiv, d.h., daß sie nicht bindegewebig eingeschlossen werden, sondern bei direktem Knochenkontakt durch die Anwesenheit des erfindungsgemäßen Ersatzteils neues Knochenwachstum möglich wird.

Durch die einfache Formbarkeit bzw. leichte Bearbeitbarkeit kann eine individuelle Formanpassung vorgenommen werden, so daß die Knochenersatzstrukturen in idealisierter Formgebung den entsprechenden natürlichen Knochen nachbilden können. Als Formkörper werden auch Granulate verstanden, die zur Ausfüllung eines Knochendefektes implantiert werden.

Die erfindungsgemäßen extrakoporal gefertigten biomimetischen Knochenstrukturen aus nicht geschäumtem Glasionomerzement eignen sich beispielsweise für folgende Einsatzgebiete:
(1) Ohr
   Äußeres Ohr
   - Ersatz für das Ohrmuschelgerüst
   Mittelohr
   - idealisierter Amboß
   - idealisierter Hammer
   - idealisierter Steigbügel
   - TORP (Total Ossicular Replacement Prosthesis)
   - PORP (Partial Ossicular Replacement Prosthesis)
   - Halbmondstruktur zur Rekonstruktion des Trommelfellrahmens
   - partieller oder totaler Ersatz der hinteren Gehörgangswand
   - Einsatz bei Mastoidobliterationen (Verschluß des Schläfenbeins).
(2) Seitliche Schädelbasis
   - Deckung eines Defekts der mittleren und hinteren Schädelgrube.
(3) Schädel
   - Ersatz bei Kalottendefekten.
(4) Frontobasis
   - Rekonstruktion knöcherner Frontobasisdefekte inklusive Stirnhöhlenhinterwand und Duraläsionen.
(5) Ersatz von Schädelknochen, insbesondere bei
   - Schädelbasisdefekten und
   - Schädeldachdefekten
   - Ersatz von Gesichtsknochendefekten im Mittelgesicht, z.B. des knöchernen Nasengerüsts, des Stirnbeins, der Stirnhöhlenvorderwand, des Nasenseptums, von Oritaboden und -dach sowie der Kiefernhöhlenvorderwand
   - allgemeiner Knochensubstanzersatz und Stabilisierung von Mittelgesichtsbrücken, wobei eine Kombination mit den herkömmlich verwendeten Platten möglich ist.
(6) Kehlkopf
   - Implantate zur Stabilisierung sowie Ersatz der Luftröhre und des Larynx.
(7) Kieferchirurgie
   - Alveolarfortsatz
   - harter Gaumen
   - Ersatz von Kiefernteilen, insbesondere im Unterkiefer
   - Ersatz von Knochendefekten, zur Stabilisierung und Osteosynthese bei Le Fort Frakturen
   - als Gesichtsknochenpolster in der plastischen Chirurgie
Glasionomerzemente bestehen im wesentlichen aus folgenden Bestandteilen:
(a) einem Glas oder Metalloxid, das durch Säurezersetzung Metallionen abgibt, welche zur Vernetzung von (b) führen,
(b) einer polymeren Polysäure, wobei die Säurefunktion eine Sulfon-, Phosphon- oder Carbonsäure ist,
(c) Wasser, sowie gegebenenfalls
(d) einem Chelatbildner.

Darüberhinaus können Stabilisatoren, Desinfektionsmittel, Pigmente, Röntgenkontrastmittel und weitere Füllstoffe vorhanden sein.

Die Glasionomerzemente werden entweder als Gemisch aus Glas und polymerer Polysäure einerseits sowie Wasser andererseits angeboten, wobei der Chelatbildner einer der beiden Komponenten zugesetzt sein kann. Ebenso ist es möglich, die polymere Polysäure in Wasser zu lösen, ggf. den Chelatbildner zuzusetzen und diese Lösung mit dem Glas anzumischen.

Als Glaspulver kommen neben den in DE-A-2 061 513 und DE-A-3 248 357 A1 genannten calcium-, magnesium- und lanthanhaltigen Glaspulvern sowie strontiumhaltigen Glaspulvern gemäß EP-A-0 241 277 auch Glaspulver mit anderen Kationen zum Einsatz. Bevorzugt verwendet werden Calcium- und/oder Strontiumfluorosilicatgläser, so daß die Aluminiumfluorosilicatglaspulver neben Sauerstoff folgende Bestandteile aufweisen können:

| Bestandteil | berechnet als | Gew.-% |
|---|---|---|
| Si | SiO₂ | 20 - 60 |
| Al | Al₂O₃ | 10 - 50 |
| Ca | CaO | 0 - 40 |
| Sr | SrO | 0 - 40 |
| F | F | 1 - 40 |
| Na | Na₂O | 0 - 10 |
| P | P₂O₅ | 0 - 10 |

Dabei müssen mindestens 1 Gew.-% CaO und/oder SrO enthalten sein. Ferner können insgesamt 0 - 20 Gew.-%, berechnet als Oxide, an B, Bi, Zn, Mg, Sn, Ti, Zr, La oder anderen 3-wertigen Lanthanoiden, K, W, Ge sowie weiteren Zusätzen enthalten sein, welche die Eigenschaften nicht beeinträchtigen und physiologisch unbedenklich sind. Durch Zusatz von 10 - 20 Gew.-% La₂O₃ können die Gläser röntgensichtbar gemacht werden.

Vorteilhaft bestehen die Pulverteilchen aus:

| | |
|---|---|
| Si als SiO₂ | 25 - 50 Gew.-% |
| Al als Al₂O₃ | 10 - 40 Gew.-% |
| Ca als CaO | 0 - 35 Gew.-% |
| Sr als SrO | 0 - 35 Gew.-% |
| F | 5 - 30 Gew.-% |
| Na als Na₂O | 0 - 8 Gew.-% |
| P als P₂O₅ | 1 - 10 Gew.-% |

Hierbei müssen mindestens 10 Gew.-% Ca (berechnet als CaO) und/oder Sr (berechnet als SrO) enthalten sein. Ferner sind 0 - 10 Gew.-% an B₂O₃, Bi₂O₃, ZnO, MgO, SnO₂, TiO₂, ZrO, La₂O₃ oder anderen Oxiden 3-wertiger Lanthanoide, K₂O, WO₃, GeO₂ sowie weiteren Zusätzen möglich, welche die Eigenschaften nicht beeinträchtigen und physiologisch unbedenklich sind.

Besonders bevorzugt verwendete Pulver enthalten:

| | |
|---|---|
| Si als SiO₂ | 25 - 45 Gew.-% |
| Al als Al₂O₃ | 20 - 40 Gew.-% |
| Ca als CaO | 10 - 30 Gew.-% |
| F | 10 - 30 Gew.-% |
| Na als Na₂O | 1 - 8 Gew.-% |
| P als P₂O₅ | 1 - 10 Gew.-% |

Beispiele der bevorzugt verwendeten Zusammensetzungen sind in der folgenden TABELLE aufgeführt:

**TABELLE**

| (Gew.-%) | | | | |
|---|---|---|---|---|
| | A | B | C | D |
| Si als SiO₂ | 35,0 | 27,6 | 29,0 | 45,4 |
| Al als Al₂O₃ | 30,4 | 26,0 | 25,1 | 35,0 |
| Ca als CaO | 14,9 | 28,8 | 24,6 | 10,1 |
| F | 17,7 | 17,0 | 23,0 | 10,3 |
| Na als Na₂O | 2,7 | 2,1 | 2,2 | 6,9 |
| P als P₂O₅ | 6,9 | 8,3 | 5,8 | 2,4 |

Die erfindungsgemäß verwendeten Glaspulverpartikel können an der Oberfläche an Calcium- oder Strontium verarmt sein, wie es für Calcium in EP-A-0 023 013 beschrieben ist.

Die erfindungsgemäß verwendeten Glaspulver weisen eine durchschnittliche Korngröße (Gewichtsmittel) von wenigstens 1 »m, bevorzugt mindestens 3 »m auf. Die durchschnittliche Korngröße (Gewichtsmittel) beträgt 1 - 20 »m, bevorzugt 3 - 15 »m, besonders bevorzugt 3 - 10 »m. Die Teilchen haben eine maximale Korngröße von 150 »m, vorzugsweise 100 »m, besonders bevorzugt 60 »m. Zur Erzielung guter mechanischer Eigenschaften ist eine nicht zu enge Korngrößenverteilung günstig, wie sie beispielsweise durch Vermahlung und Absiebung der Grobanteile erreicht wird.

Die als Bestandteil (b) einzusetzenden polymeren Polysäuren können auch die bei Herstellung von Glasionomerzementen bekannten Polycarbonsäuren, z.B. Polymaleinsäure, Polyacrylsäure, Polyitaconsäure sowie Gemische davon oder Copolymere, insbesondere die aus EP-A-0 024 056 bekannten Maleinsäure-Acrylsäure-Copolymere und/oder Acrylsäure-Itakonsäure-Copolymere sein. Das mittlere Molekulargewicht der einzusetzenden Polycarbonsäuren beträgt mehr als 500. Vorteilhaft ist ein mittleres Molekulargewicht von 1.000 - 20.000, besonders bevorzugt sind 3.000 - 10.000. Die Polycarbonsäure wird vozugsweise in Konzentrationen von 5 - 50 Gew.-%, bezogen auf den Bestandteil (a), eingesetzt.

Die bekannten chelatbildenden Zusätze können auch im erfindungsgemäßen Glasionomerzement als Bestandteil (d) verwendet werden (vgl. DE-A-2 319 715). Vorzugsweise wird als Chelatbildner Weinsäure eingesetzt.

### Beispiel 1

250 Gew.-teile eines Calciumaluminiumfluorosilicatglaspulvers der Zusammensetzung A der obigen TABELLE werden mit 100 Gew.-teilen einer Lösung aus 37 g eines Copolymeren (1:1) aus Acrylsäure und Maleinsäure, 9 g Weinsäure und 54 g Wasser vermischt.

Aus dem pastösen Material läßt sich manuell eine Knochenstruktur zum Ersatz einer hinteren Gehörwand formen. Nach ca. 10 Minuten ist das Ersatzteil völlig erhärtet und kann mit frisch angemischtem, noch plastischem Zement in situ verbracht werden.

Drei Wochen nach der Operation ist der Knochenersatz völlig problemlos inkorporiert. Der Verbund ist fugenlos.

### Beispiel 2

Von dem angemischten Material nach Beispiel 1 werden Formkörper mit den Ausmaßen 15 x 20 x 5 mm hergestellt und in die linke Tibia eines Pavians implantiert. Auf den Röntgenaufnahmen unterscheidet sich das Implantat nicht vom Kochenmaterial. Nach zwei Wochen zeigt sich am Rand des Implantats eine ausgeprägte Knochenbildungsaktivität und nach weiteren vier Wochen ist die Stelle nicht mehr vom umgebenden Knochenmaterial zu unterscheiden, da das Implantat vollkommen von neugebildetem Knochenmaterial umgeben ist.

### Beispiel 3

Aus dem angemischten Zement nach Beispiel 1 wird nach vorliegender Zeichnung ein idealisiertes Gehörknöchelchen (Amboß) mit abgerundeter Form hergestellt. Runde Formen sind biokompatibler als scharfkantige wie bei einem Keramik-Formkörper. Die Gefahr, das Trommelfell zu durchstoßen ist kleiner, Epithelzellen wachsen bevorzugt über runde Formen.

### Beispiel 4

Der Formkörper nach Beispiel 2 wird mit in der HNO-Chirurgie üblichen Fräsinstrumenten bearbeitet und daraus ein idealisierter Amboß geformt (siehe Zeichnung). Die Bearbeitung ist leicht und ohne Rißbildung, Abplatzungen oder Bruch im gewünschten Formkörper möglich.

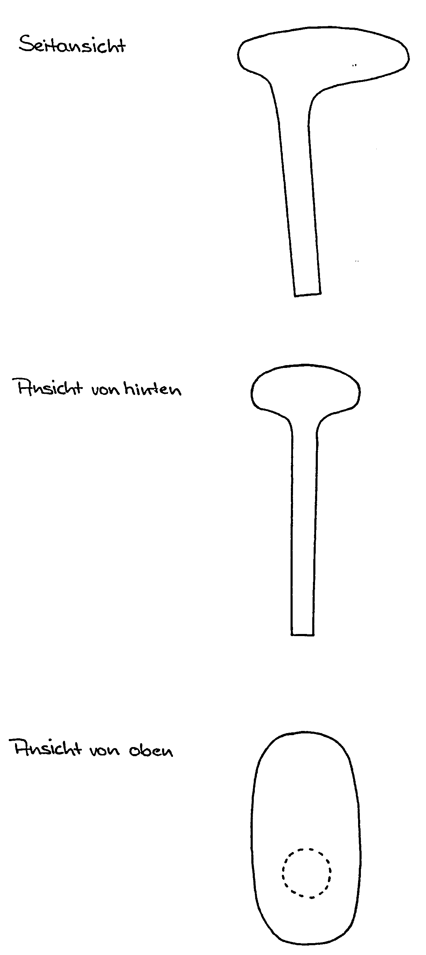

## Patentansprüche (Patentansprüche für folgende(n) Vertragsstaat(en): AT, CH, DE, FR, GB, IT, LI, SE)

1. Verwendung eines, insbesondere nicht geschäumten, Glasionomerzements für die extrakorporale Herstellung von Knochenstrukturen oder Knochenersatzteilen zur Implantation in den Körper, wobei der Glasionomerzement die folgenden Bestandteile enthält :
(a) ein Aluminiumfluorsilikatglaspulver, das durch Säurezersetzung Metallionen abgibt, welche zur Vernetzung von (b) führen,
(b) eine polymere Polysäure, wobei die Säurefunktion eine Sulfon-, Phosphon- oder Carbonsäure ist, sowie
(c) Wasser.

2. Verwendung nach Anspruch 1 zur Herstellung von Prothesen für Ohr-, Schädel-, Kehlkopf- oder Kieferknochen.

3. Knochenersatzteil, dadurch gekennzeichnet, daß es aus einem in Anspruch 1 definierten nicht geschäumten Glasionomerzement besteht.

4. Knochenersatzteil nach Anspruch 3, dadurch gekennzeichnet, daß der Glasionomerzement folgende Bestandteile enthält:
(a) ein Calcium- und/oder Strontium-Aluminiumfluorosilicatglaspulver mit 20 - 60 Gew.-% SiO₂, 10 - 50 Gew.-% Al₂O₃, 0 - 40 Gew.-% CaO, 0 - 40 Gew.-% SrO, 1 - 40 Gew.-% F, 0 - 10 Gew.-% Na₂O und 0 - 10 Gew.-% P₂O₅, wobei mindestens 1 Gew.-% CaO und/oder SrO enthalten ist,
(b) eine Polycarbonsäure mit einem mittleren Moleculargewicht von 1.000 - 20.000, in einer Konzentration von 5 - 50 Gew.-%, bezogen auf den Bestandteil (a),
(c) Wasser und gegebenenfalls
(d) Weinsäure als Chelatbildner.

5. Knochenersatzteil nach Anspruch 4, dadurch gekennzeichnet, daß der Bestandteil (a) 25 - 50 Gew.-% SiO₂, 10 - 40 Gew.-% Al₂O₃, 0 - 35 Gew.-% CaO, 0 - 35 Gew.-% SrO, 5 - 30 Gew.-% F, 0 - 8 Gew.-% Na₂O, und 1 - 10 Gew.-% P₂O₅ enthält, wobei mindestens 10 Gew.-% CaO und/oder SrO enthalten sind.

6. Knochenersatzteil nach Anspruch 5, dadurch gekennzeichnet, daß der Bestandteil (a) 25 - 45 Gew.-% SiO₂, 20 - 40 Gew.-% Al₂O₃, 10 - 30 Gew.-% CaO, 10 - 30 Gew.-% F, 1 - 8 Gew.-% Na₂O, und 1 - 10 Gew.-% P₂O₅ enthält.

7. Knochenersatzteil nach einem der Ansprüche 4 bis 6, dadurch gekennzeichnet, daß die durchschnittliche Korngröße des Glaspulvers 1 - 20 »m bei einer maximalen Korngröße von 150 »m, vorzugsweise 3 - 10 »m bei einer maximalen Korngröße von 60 »m beträgt.

8. Verfahren zur Herstellung eines Knochenersatzteils, dadurch gekennzeichnet, daß aus einem in Anspruch 1 definierten nicht geschäumten Glasionomerzement ein Formkörpor vorgefertigt und dieser durch spanabhebende Bearbeitung, insbesondere Schleifen und/oder Fräsen, auf die Form des zu ersetzenden Knochenteils gebracht wird.

9. Verfahren nach Anspruch 8, dadurch gekennzeichnet, daß der Formkörper mit einer dem vorgesehenen Einsatzzweck im wesentlichen entsprechenden Form und Größe vorgefertigt wird.

## Patentansprüche (Patentansprüche für folgende(n) Vertragsstaat(en): ES)

1. Verfahren zur extrakorporalen Herstellung von Knochenstrukturen oder Knochenersatzteilen zur Implantation in den Körper durch Verformen und Härten eines nicht geschäumten Glasionomerzements, der folgende Bestandteile enthält :
(a) ein Aluminiumfluorsilikatglaspulver, das durch Säurezersetzung Metallionen abgibt, welche zur Vernetzung von (b) führen,
(b) eine polymere Polysäure, wobei die Säurefunktion eine Sulfon-, Phosphon- oder Carbonsäure ist, sowie
(c) Wasser.

2. Verfahren nach Anspruch 1 zur Herstellung von Prothesen für Ohr-, Schädel-, Kehlkopf- oder Kieferknochen.

3. Knochenersatzteil, dadurch gekennzeichnet, daß es aus einem in Anspruch 1 definierten nicht geschäumten Glasionomerzement besteht.

4. Knochenersatzteil nach Anspruch 3, dadurch gekennzeichnet, daß der Glasionomerzement folgende Bestandteile enthält:
(a) ein Calcium- und/oder Strontium-Aluminiumfluorosilicatglaspulver mit 20 - 60 Gew.-% SiO₂, 10 - 50 Gew.-% Al₂O₃, 0 - 40 Gew.-% CaO, 0 - 40 Gew.-% SrO, 1 - 40 Gew.-% F, 0 - 10 Gew.-% Na₂O und 0 - 10 Gew.-% P₂O₅, wobei mindestens 1 Gew.-% CaO und/oder SrO enthalten ist,
(b) eine Polycarbonsäure mit einem mittleren Moleculargewicht von 1.000 - 20.000, in einer Konzentration von 5 - 50 Gew.-%, bezogen auf den Bestandteil (a),
(c) Wasser und gegebenenfalls
(d) Weinsäure als Chelatbildner.

5. Knochenersatzteil nach Anspruch 4, dadurch gekennzeichnet, daß der Bestandteil (a) 25 - 50 Gew.-% SiO₂, 10 - 40 Gew.-% Al₂O₃, 0 - 35 Gew.-% CaO, 0 - 35 Gew.-% SrO, 5 - 30 Gew.-% F, 0 - 8 Gew.-% Na₂O, und 1 - 10 Gew.-% P₂O₅ enthält, wobei mindestens 10 Gew.-% CaO und/oder SrO enthalten sind.

6. Knochenersatzteil nach Anspruch 5, dadurch gekennzeichnet, daß der Bestandteil (a) 25 - 45 Gew.-% SiO₂, 20 - 40 Gew.-% Al₂O₃, 10 - 30 Gew.-% CaO, 10 - 30 Gew.-% F, 1 - 8 Gew.-% Na₂O, und 1 - 10 Gew.-% P₂O₅ enthält.

7. Knochenersatzteil nach einem der Ansprüche 4 bis 6, dadurch gekennzeichnet, daß die durchschnittliche Korngröße des Glaspulvers 1 - 20 »m bei einer maximalen Korngröße von 150 »m, vorzugsweise 3 - 10 »m bei einer maximalen Korngröße von 60 »m beträgt.

8. Verfahren zur Herstellung eines Knochenersatzteils, dadurch gekennzeichnet, daß aus einem in Anspruch 1 definierten nicht geschäumten Glasionomerzement ein Formkörper vorgefertigt und dieser durch spanabhebende Bearbeitung, insbesondere Schleifen und/oder Fräsen, auf die Form des zu ersetzenden Knochenteils gebracht wird.

9. Verfahren nach Anspruch 8, dadurch gekennzeichnet, daß der Formkörper mit einer dem vorgesehenen Einsatzzweck im wesentlichen entsprechenden Form und Größe vorgefertigt wird.

## Claims (Claims for the following Contracting State(s): AT, CH, DE, FR, GB, IT, LI, SE)

1. The use of a glass ionomer cement, particularly a non-foamed glass-ionomer cement, for the extra-corporeal production of bone structures or bone-replacement parts for implantation in the body, wherein the glass ionomer cement comprises the following components :
(a) an aluminum fluorosilicate glass powder which releases metal ions upon decomposition by acid, said metal ions causing crosslinking of (b),
(b) a polymer polyacid having a sulfonic, phosphonic or carboxylic acid functional group, and
(c) water.

2. The use according to claim 1 for the production of prostheses for ear, cranium, larynx or jaw-bones.

3. Bone-replacement part characterised in that it consists of a non-foamed glass ionomer cement as defined in claim 1.

4. Bone-replacement part according to claim 3, characterised in that the glass ionomer cement comprises the following components :
(a) a calcium and/or strontium aluminum fluorosilicate glass powder comprising 20 - 60 wt-% SiO₂, 10 - 50 wt-% Al₂O₃, 0 - 40 wt-% CaO, 0 - 40 wt-% SrO, 1 - 40 wt-% F, 0 - 10 wt-% Na₂O and 0 - 10 wt-% P₂O₅, wherein at least 1 wt-% CaO and/or SrO is present,
(b) a polycarboxylic acid having an average molecular weight of from 1,000 to 20,000 in a concentration of 5 - 50 wt-%, based on component (a),
(c) water and optionally
(d) tartaric acid as a chelating agent.

5. Bone-replacement part according to claim 4, characterised in that component (a) comprises 25 - 50 wt-% SiO₂, 10 - 40 wt-% Al₂O₃, 0 - 35 wt-% CaO, 0 - 35 wt-% SrO, 5 - 30 wt-% F, 0 - 8 wt-% Na₂O and 1 - 10 wt-% P₂O₅, wherein at least 10 wt-% CaO and/or SrO are present.

6. Bone-replacement part according to claim 5 characterised in that component (a) comprises 25 - 45 wt-% SiO₂, 20 - 40 wt-% Al₂O₃, 10 - 30 wt-% CaO, 10 - 30 wt-% F, 1 - 8 wt-% Na₂O and 1 - 10 wt-% P₂O₅.

7. Bone-replacement part according to any of the claims 4 to 6, characterised in that the average particle size of the glass powder is 1 - 20 »m at a maximum particle size of 150 »m, and preferably 3 - 10 »m at a maximum particle size of 60 »m.

8. A process for producing a bone-replacement part, characterised in that a shaped body is preformed from a non-foamed glass ionomer cement as defined in claim 1 and the said shaped body is formed into the shape of the bone part to be replaced by a cutting process, specifically by grinding and/or milling.

9. The process according to claim 8, characterised in that the shaped body is prefabricated in a shape and size substantially corresponding to the intended application.

## Claims (Claims for the following Contracting State(s): ES)

1. A process for the extra-corporeal production of bone structures or bone-replacement parts for implantation in the body by shaping and curing a non-foamed glass ionomer cement comprising the following components :
(a) an aluminum fluorosilicate glass powder which releases metal ions upon decomposition by acid, said metal ions causing crosslinking of (b),
(b) a polymer polyacid having a sulfonic, phosphonic or carboxylic acid functional group, and
(c) water.

2. The process according to claim 1 for the production of prostheses for ear, cranium, larynx or jaw-bones.

3. Bone-replacement part characterised in that it consists of a non-foamed glass ionomer cement as defined in claim 1.

4. Bone-replacement part according to claim 3, characterised in that the glass ionomer cement comprises the following components :
(a) a calcium and/or strontium aluminum fluorosilicate glass powder comprising 20 - 60 wt-% SiO₂, 10 - 50 wt-% Al₂O₃, 0 - 40 wt-% CaO, 0 - 40 wt-% SrO, 1 - 40 wt-% F, 0 - 10 wt-% Na₂O and 0 - 10 wt-% P₂O₅, wherein at least 1 wt-% CaO and/or SrO is present,
(b) a polycarboxylic acid having an average molecular weight of from 1,000 to 20,000 in a concentration of 5 - 50 wt-%, based on component (a),
(c) water and optionally
(d) tartaric acid as a chelating agent.

5. Bone-replacement part according to claim 4, characterised in that component (a) comprises 25 - 50 wt-% SiO₂, 10 - 40 wt-% Al₂O₃, 0 - 35 wt-% CaO, 0 - 35 wt-% SrO, 5 - 30 wt-% F, 0 - 8 wt-% Na₂O and 1 - 10 wt-% P₂O₅, wherein at least 10 wt-% CaO and/or SrO are present.

6. Bone-replacement part according to claim 5 characterised in that component (a) comprises 25 - 45 wt-% SiO₂, 20 - 40 wt-% Al₂O₃, 10 - 30 wt-% CaO, 10 - 30 wt-% F, 1 - 8 wt-% Na₂O and 1 - 10 wt-% P₂O₅.

7. Bone-replacement part according to any of the claims 4 to 6, characterised in that the average particle size of the glass powder is 1 - 20 »m at a maximum particle size of 150 »m, and preferably 3 - 10 »m at a maximum particle size of 60 »m.

8. A process for producing a bone-replacement part, characterised in that a shaped body is preformed from a non-foamed glass ionomer cement as defined in claim 1 and the said shaped body is formed into the shape of the bone part to be replaced by a cutting process, specifically by grinding and/or milling.

9. The process according to claim 8, characterised in that the shaped body is prefabricated in a shape and size substantially corresponding to the intended application.

## Revendications (Revendications pour l'(les) Etat(s) contractant(s) suivant(s): AT, CH, DE, FR, GB, IT, LI, SE)

1. Utilisation d'un ciment de verre ionomère, notamment non cellulaire, pour la fabrication extracorporelle de structures ou de prothèses osseuses pour l'implantation dans le corps, le ciment de verre ionomère contenant les constituants suivants :
(a) une poudre de verre de fluorosilicate d'aluminium qui cède, par décomposition d'acide, des ions métalliques qui conduisent à la réticulation de (b),
(b) un polyacide polymère, la fonction acide étant un acide sulfonique, phosphonique ou carboxylique, et
(c) de l'eau.

2. Utilisation selon la revendication 1, pour la réalisation de prothèses des os de l'oreille, du crâne, du larynx ou de la mâchoire.

3. Prothèse osseuse, caractérisée en ce qu'elle est constituée d'un ciment de verre ionomère non cellulaire défini dans la revendication 1.

4. Prothèse osseuse selon la revendication 3, caractérisée en ce que le ciment de verre ionomère contient les constituants suivants :
(a) une poudre de verre de fluorosilicate d'aluminium et de calcium et/ou de strontium avec 20 à 60 % en poids de SiO₂, 10 à 50 % en poids de Al₂O₃, 0 à 40 % en poids de CaO, 0 à 40 % en poids de SrO, 1 à 40 % en poids de F, 0 à 10 % en poids de Na₂O et 0 à 10 % en poids de P₂O₅, avec une teneur d'au moins 1 % en poids de CaO et/ou de SrO,
(b) un acide polycarboxylique avec un poids moléculaire moyen de 1.000 à 20.000, dans une concentration de 5 à 50 % en poids, rapportée au constituant (a),
(c) de l'eau et éventuellement
(d) de l'acide tartrique comme chélateur.

5. Prothèse osseuse selon la revendication 4, caractérisée en ce que le constituant (a) contient 25 à 50 % en poids de SiO₂, 10 à 40 % en poids de Al₂O₃, 0 à 35 % en poids de CaO, 0 à 35 % en poids de SrO, 5 à 30 % en poids de F, 0 à 8 % en poids de Na₂O et 1 à 10 % en poids de P₂O₅, avec une teneur d'au moins 10 % en poids de CaO et/ou de SrO.

6. Prothèse osseuse selon la revendication 5, caractérisée en ce que le constituant (a) contient 25 à 45 % en poids de SiO₂, 20 à 40 % en poids de Al₂O₃, 10 à 30 % en poids de CaO, 10 à 30 % en poids de F, 1 à 8 % en poids de Na₂O et 1 à 10 % en poids de P₂O₅.

7. Prothèse osseuse selon l'une quelconque des revendications 4 à 6, caractérisée en ce que la grosseur de grain moyenne de la poudre de verre est de 1 à 20 »m pour une grosseur de grain maximale de 150 »m, de préférence de 3 à 10 »m pour une grosseur de grain maximale de 60 »m.

8. Procédé de fabrication d'une prothèse osseuse, caractérisé en ce qu'un corps moulé est préfabriqué à partir d'un ciment de verre ionomère non cellulaire défini dans la revendication 1 et que ledit corps moulé est amené, par enlèvement de copeaux, notamment par meulage et/ou fraisage, à la forme de l'élément osseux à remplacer.

9. Procédé selon la revendication 8, caractérisé en ce que le corps moulé est préfabriqué avec une forme et une dimension qui correspondent sensiblement à l'utilisation prévue.

## Revendications (Revendications pour l'(les) Etat(s) contractant(s) suivant(s): ES)

1. Procédé de fabrication extracorporelle de structures ou prothèses osseuses pour l'implantation dans le corps par déformation et durcissement d'un ciment de verre ionomère non cellulaire qui contient les constituants suivants :
(a) une poudre de verre de fluorosilicate d'aluminium qui cède, par décomposition d'acide, des ions métalliques qui conduisent à la réticulation de (b),
(b) un polyacide polymère, la fonction acide étant un acide sulfonique, phosphonique ou carboxylique, et
(c) de l'eau.

2. Procédé selon le revendication 1 pour la réalisation de prothèses des os de l'oreille, du crâne, du larynx ou de la mâchoire.

3. Prothèse osseuse, caractérisée en ce qu'elle est constituée d'un ciment de verre ionomère non cellulaire défini dans la revendication 1.

4. Prothèse osseuse selon la revendication 3, caractérisée en ce que le ciment de verre ionomère contient les constituants suivants :
(a) une poudre de verre de fluorosilicate d'aluminium et de calcium et/ou de strontium avec 20 à 60 % en poids de. SiO₂, 10 à 50 % en poids de Al₂O₃, 0 à 40 % en poids de CeO, 0 à 40 % en poids de SrO, 1 à 40 % en poids de F, 0 à 10 % en poids de Na₂O et 0 à 10 % en poids de P₂O₅, avec une teneur d'au moins 1 % en poids de CaO et/ou de SrO,
(b) un acide polycarboxylique avec un poids moléculaire moyen de 1.000 à 20.000, dans une concentration de 5 à 50 % en poids, rapportée au constituant (a),
(c) de l'eau et éventuellement
(d) de l'acide tartrique comme chélateur.

5. Prothèse osseuse selon la revendication 4, caractérisée en ce que le constituant (a) contient 25 à 50 % en poids de SiO₂, 10 à 40 % en poids de Al₂O₃, 0 à 35 % en poids de CaO, 0 à 35 % en poids de SrO, 5 à 30 % en poids de F, 0 à 8 % en poids de Na₂O et 1 à 10 % en poids de P₂O₅, avec une teneur d'au moins 10 % en poids de CaO et/ou de SrO.

6. Prothèse osseuse selon la revendication 5, caractérisée en ce que le constituant (a) contient 25 à 45 % en poids de SiO₂, 20 à 40 % en poids de Al₂O₃, 10 à 30 % en poids de CaO, 10 à 30 % en poids de F, 1 à 8 % en poids de Na₂O et 1 à 10 % en poids de P₂O₅.

7. Prothèse osseuse selon l'une quelconque des revendications 4 à 6, caractérisée en ce que la grosseur de grain moyenne de la poudre de verre est de 1 à 20 »m pour une grosseur de grain maximale de 150 »m, de préférence de 3 à 10 »m pour une grosseur de grain maximale de 60 »m.

8. Procédé de fabrication d'une prothèse osseuse, caractérisé en ce qu'un corps moulé est préfabriqué à partir d'un ciment de verre ionomère non cellulaire défini dans la revendication 1 et que ledit corps moulé est amené, par enlèvement de copeaux, notamment par meulage et/ou fraisage, à la forme de l'élément osseux à remplacer.

9. Procédé selon la revendication 8, caractérisé en ce que le corps moulé est préfabriqué avec une forme et une dimension qui correspondent sensiblement à l'utilisation prévue.
